# EUROPÄISCHE PATENTANMELDUNG

(11) **EP 1 087 170 A2**
(43) Veröffentlichungstag der Anmeldung: **28.03.2001**
(21) Anmeldenummer: 00102002.3
(22) Anmeldetag: 02.02.2000
(51) Int. Cl.: F16L 41/02

(54) **Vorrichtung zum Anschlusss von Geräten und Anlagen an Flüssigkeitsleitungen**

(30) Priorität: 22.09.1999 DE 19945496
(71) Anmelder: GML KfH Gesellschaft für Medizintechnik und Logistik m.b.H., 63263 Neu-Isenburg (DE)
(72) Erfinder: Kurina, Hans, 48268 Greven (DE); Kaupa, Paul, 64331 Weiterstadt (DE)
(74) Vertreter: Flosdorff, Jürgen, Dr.

(57) **Zusammenfassung**

Die Vorrichtung zum Anschluß von Geräten und Anlagen an Flüssigkeitsleitungen (6,7) enthält einen außerhalb der Flüssigkeitsleitung verbleibenden Anschlußnippel (2) und einen damit verbundenen, ins Innere der Flüssigkeitsleitung ragenden rohrförmigen Abschnitt (4). Der Endbereich (9) der Wand des rohrförmigen Abschnitts ist mit Löchern (10) versehen, so daß der in die Flüssigkeitsleitung hineinragende Teil des rohrförmigen Abschnitts stets von der Flüssigkeit umspült und durchgespült wird, wodurch in diesem Bereich keine organischen oder anorganischen Ablagerungen bilden können.

## Beschreibung

Die Erfindung betrifft eine Vorrichtung zum Anschluß von Geräten und Anlagen an Flüssigkeitsleitungen gemäß dem Oberbegriff des Patentanspruchs 1. Die Leitungen können dabei beispielsweise mit aufbereitetem Wasser, flüssigen Lebensmitteln, Arzneimittel oder Medikalprodukten gefüllt sein, ohne daß die Erfindung hierauf beschränkt ist.

Beim Anschluß von Geräten und Anlagen an Leitungen werden im allgemeinen Anschlüsse aus Kunststoff oder Edelstahl verwendet, die nicht auf die Durchmesser der Leitung abgestimmt sind. Für den Anschluß an die Leitungen werden meist T-Anschlußstücke eingesetzt, deren Mittelteile hinsichtlich der Anschlußlänge nicht mit dem Gewindeteil eines Anschlußnippels abgestimmt sind. Hierdurch kommt es zur Bildung von Toträumen in den T-Anschlüssen, wo sich Ablagerungen bilden können.

Organische Ablagerungen, wie Bakterien und deren Ausscheidungsprodukt Endotoxine können, wenn sie in die Leitung oder angeschlossenen Geräte und Anlagen gespült werden, hergestellte Produkte unbrauchbar machen oder beim Anschluß von Geräten für die Behandlung von Patienten diese gefährden.

Der vorliegenden Erfindung liegt die Aufgabe zugrunde, eine Anschlußvorrichtung der betrachteten Art so auszubilden, daß die Entstehung von organischen und anorganischen Ablagerungen in ihrem Bereich weitestgehend vermieden ist.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, daß die Anschlußvorrichtung einen Anschlußnippel, der in der Einbaulage außerhalb der Flüssigkeitsleitung verbleibt, und einen eine Bohrung durch die Wand der Flüssigkeitsleitung durchgreifenden rohrförmigen Abschnitt aufweist, von dem zumindest der Endbereich innerhalb der Flüssigkeitsleitung angeordnet wird, und daß der Endbereich der Wand des rohrförmigen Abschnitts mit Löchern versehen ist.

Durch diese erfindungsgemäße Ausbildung ist gewährleistet, daß der in die Flüssigkeitsleitung hineinragende Teil des rohrförmigen Abschnitts so von der Flüssigkeit umspült und durchgespült wird, daß ein totraumfreier Anschluß der Geräte und Anlagen an die Leitung gewährleistet ist. Durch die eine Umspülung gewährleistenden Bohrungen ist zuverlässig verhindert, daß sich organische und anorganische Ablagerungen bilden können.

In weiteren Einzelheiten wird vorgeschlagen, daß die Löcher gleichmäßig über den Umfang des rohrförmigen Abschnitts verteilt ausgebildet sind, wobei diese Löcher vorzugsweise eine Kreisform haben. Es sind bevorzugt ca. 4 bis 8 Löcher vorgesehen, wobei der Zwischenraum zwischen den Löchern etwa deren Durchmesser entsprechen kann. Die Erfindung ist aber hierauf nicht beschränkt, sondern es können auch kleinere Löcher in größerer Anzahl und beispielsweise mehr als eine umlaufende Reihe von Löchern vorgesehen sein.

Zweckmäßigerweise ist der rohrförmige Abschnitt der Anschlußvorrichtung mit einem Außengewinde versehen, so daß er in die Bohrung der Flüssigkeitsleitung eingeschraubt werden kann. Die Erfindung ist hierauf nicht beschränkt, beispielsweise kann der rohrförmige Abschnitt auch durch die Wand der Flüssigkeitsleitung hindurchgesteckt und auf geeignete Weise abgedichtet sein. Bevorzugt ist aber eine Schraubverbindung.

Zweckmäßigerweise erstreckt sich das Außengewinde bis zu dem gelochten Endabschnitt, der eine glatte Außenwand haben sollte, um die Entstehung von Ablagerungen an der Wand auszuschließen.

In einer bevorzugten Ausführungsform der Erfindung weist die Anschlußvorrichtung zwischen dem Anschlußnippel und dem rohrförmigen Abschnitt eine ringförmige Scheibe auf, die an die Außenwand der Flüssigkeitsleitung festziehbar ist. In dieser Ausführungsform ist die Anschlußvorrichtung eine selbstdichtende Kupplung, die beispielsweise für Dialysegeräte bestens geeignet ist.

Durch Variation der Form des Anschlußnippels können beliebige Konstellationen erreicht werden.

Weitere Einzelheiten der Erfindung ergeben sich aus der nachfolgenden Beschreibung einer bevorzugten Ausführungsform der Erfindung sowie anhand der Zeichnung. Dabei zeigen:

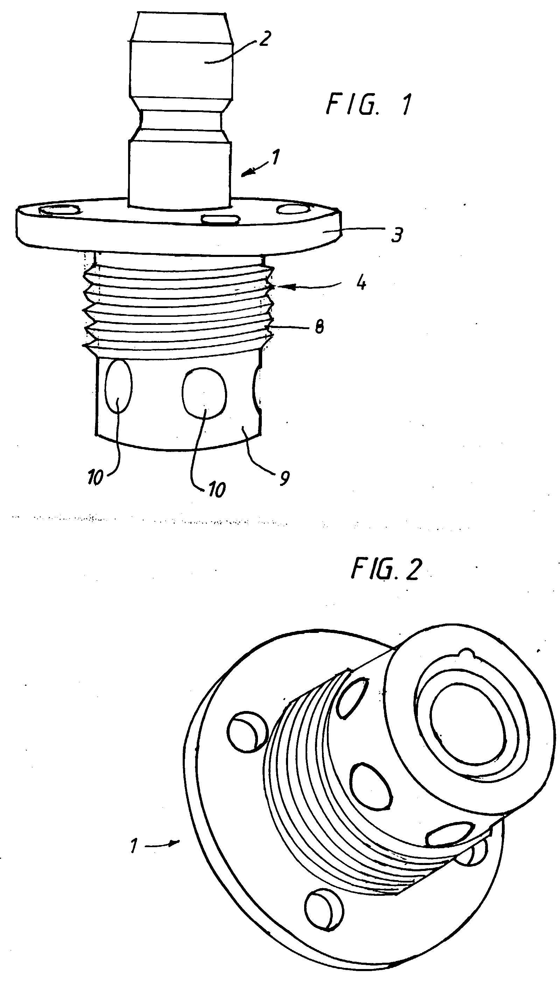

- Abb. 1: eine Seitenansicht einer erfindungsgemäßen Anschlußvorrichtung;
- Abb. 2: eine perspektivische Ansicht der Anschlußvorrichtung;
- Abb. 3: eine Aufsicht auf einen Abschnitt einer Flüssigkeitsleitung mit eingebauter Anschlußvorrichtung;
- Abb. 4: eine Stirnansicht der Anordnung gemäß Abb. 3.

Die Anschlußvorrichtung 1 ist einstückig ausgebildet und enthält einen Anschlußnippel 2, eine sich im rechten Winkel dazu erstreckende ringförmige Scheibe 3 und einen anschließenden rohrförmigen Abschnitt 4, der einen erheblich größeren Durchmesser hat als der Anschlußnippel 2.

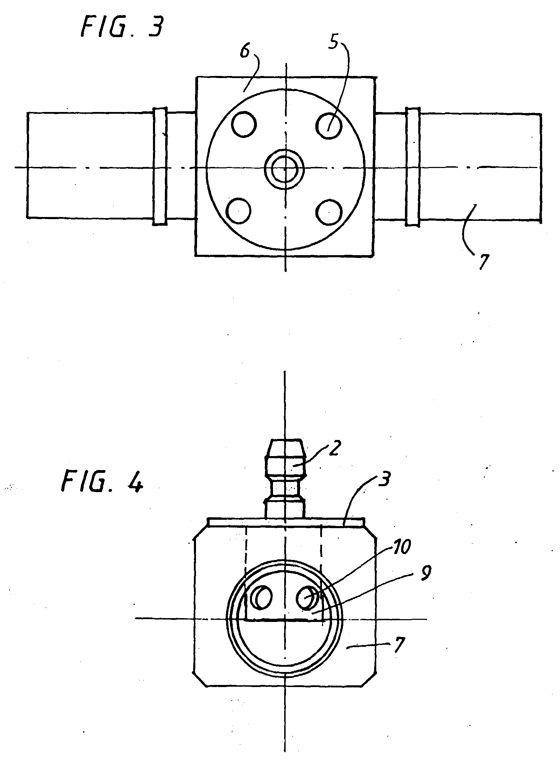

Die ringförmige Scheibe ist mit vier gleichmäßig über den Umfang verteilten Bohrungen 5 (Abb. 3) versehen, durch die Schrauben zur Befestigung der Anschlußvorrichtung an einem etwa würfelförmigen Zwischenstück 6 einer Flüssigkeitsleitung 7 hindurchgreifen können.

Der rohrförmige Abschnitt 4 enthält ein Außengewinde 8, das sich von der ringförmigen Scheibe 3 bis zu einem Endbereich 9 des rohrförmigen Abschnitts 4 erstreckt, dessen Wand gleichmäßig über den Umfang verteilte Löcher 10 enthält.

Wie Abb. 4 zeigt, befindet sich in der Einbaulage der Anschlußvorrichtung 1 der gelochte Endabschnitt 9 des rohrförmigen Abschnitts 4 innerhalb der Flüssigkeitsleitung 7. Die Löcher bzw. Bohrungen 10 stellen sicher, daß der in die Flüssigkeitsleitung 7 ragende Teil der Anschlußvorrichtung umspült bzw. durchgespült wird, wodurch verhindert wird, daß sich in diesem Bereich organische oder anorganische Ablagerungen bilden.

## Patentansprüche

1. Vorrichtung zum Anschluß von Geräten und Anlagen an Flüssigkeitsleitungen (6, 7),
**dadurch gekennzeichnet**,
daß die Anschlußvorrichtung (1) einen außerhalb der Flüssigkeitsleitung (6,7) verbleibenden Anschlußnippel (2) und einen damit verbundenen, eine Bohrung durch die Wand der Flüssigkeitsleitung (6, 7) durchgreifenden rohrförmigen Abschnitt (4) aufweist, und daß der Endbereich (9) der Wand des rohrförmigen Abschnitts (4) mit Löchern (10) versehen ist.

2. Vorrichtung nach Anspruch 1,
dadurch gekennzeichnet, daß die Löcher (10) gleichmäßig über den Umfang des rohrförmigen Abschnitts (4) verteilt ausgebildet sind.

3. Vorrichtung nach Anspruch 1 oder 2,
dadurch gekennzeichnet, daß der rohrförmige Abschnitt (4) mit einem Außengewinde (8) versehen ist.

4. Vorrichtung nach einem der Ansprüche 1 bis 3,
dadurch gekennzeichnet, daß sich das Außengewinde (8) im wesentlichen bis zu dem gelochten Endabschnitt (9) erstreckt.

5. Vorrichtung nach einem der Ansprüche 1 bis 4,
dadurch gekennzeichnet, daß der gelochte Endabschnitt (9) eine glatte Außenwand hat.

6. Vorrichtung nach einem der Ansprüche 1 bis 5,
dadurch gekennzeichnet, daß zwischen dem Anschlußnippel (2) und dem rohrförmigen Abschnitt (4) eine ringförmige Scheibe (3) ausgebildet ist, die an die Außenwand der Flüssigkeitsleitung (6) festziehbar ist.
